# EUROPÄISCHE PATENTSCHRIFT

(11) **EP 1 276 528 B1**
(45) Veröffentlichungstag und Bekanntmachung des Hinweises auf die Patenterteilung: **10.11.2004**
(21) Anmeldenummer: 01914944.2
(22) Anmeldetag: 03.04.2001
(51) Int. Cl.: A61M 5/315, A61M 5/20

(54) **VORRICHTUNG ZUR DOSIERTEN VERABREICHUNG EINES INJIZIERBAREN PRODUKTS**
DEVICE FOR THE DOSED ADMINISTRATION OF AN INJECTABLE PRODUCT
DISPOSITIF POUR L'ADMINISTRATION DOSEE D'UN PRODUIT INJECTABLE

(30) Priorität: 17.04.2000 DE 10018924
(43) Veröffentlichungstag der Anmeldung: 22.01.2003
(73) Patentinhaber: TecPharma Licensing AG, 3401 Burgdorf (CH)
(72) Erfinder: BALLY, Christoph, F-77600 Bussy St Georges (FR); JOST, Stefan, CH-3065 Bolligen (CH)
(86) Internationale Anmeldenummer: PCT/CH2001/000212
(87) Internationale Veröffentlichungsnummer: WO 2001/078811

(56) Entgegenhaltungen:
- CH-A- 364 320
- CH-A- 682 722
- FR-A- 1 528 444
- US-A- 2 750 943
- US-A- 3 504 673
- US-A- 5 865 804
- US-A- 5 975 355

## Beschreibung

Die Erfindung betrifft eine Vorrichtung zur dosierten Verabreichung eines injizierbaren Produkts.

Injizierbare Produkte, insbesondere Medikamentenfluide, bedürfen häufig einer dosierten Verabreichung. Eine mechanische und/oder elektronische Dosierung ist oft kompliziert und treibt den Preis der Vorrichtung. Insbesondere bei manueller Dosierung können ferner Dosierfehler auftreten. Um Dosierfehler zu vermeiden, muss der Verwender mit gefühlvollem Krafteinsatz dosieren.

Die Dokumente US-A-2 750 943, US-A-3 504 673, CH-A-364320 und US-A-5 865 804 offenbaren Vorrichtungen mit mechanischer Dosierung.

Die Erfindung hat es sich zur Aufgabe gemacht, eine Vorrichtung zur dosierten Verabreichung eines injizierbaren Produkts zu schaffen, welche die Sicherheit erhöht, dass eine gewünschte Dosis des Produkts bei einer Dosierung auch tatsächlich eingestellt wird, wobei eine kostengünstige und betriebssichere Lösung angestrebt wird. Vorzugsweise ist eine einfache, manuelle Dosierung möglich.

Die Aufgabe wird durch den Gegenstand von Anspruch 1 gelöst. Eine Vorrichtung laut Oberbegriff von Anspruch 1 ist in US-A-2 750 943 offenbart.

Eine erfindungsgemäße Vorrichtung zur dosierten Verabreichung eines injizierbaren Produkts umfasst ein Gehäuse mit einem Reservoir, einen Kolben, eine Ausschütteinrichtung und eine Dosiereinrichtung. Das Reservoir wird vorzugsweise von einer Ampulle gebildet, in der sich ein injizierbares Produkt befindet. Der Kolben verdrängt bei einer Verschiebung in eine Vorschubrichtung auf einen Auslass des Reservoirs zu Produkt aus dem Reservoir. Eine Dosierung erfolgt durch Einstellung einer Weglänge, um die der Kolben bei einem Betätigungsvorgang maximal verschoben werden kann.

Die Ausschütteinrichtung drückt mit einem Vorschubelement den Kolben um die eingestellte Weglänge in die Vorschubrichtung. Die Dosiereinrichtung umfasst mehrere Dosierkörper und ein Dosierelement. Die Weglänge wird durch die Anzahl und Dicke oder Dicken der Dosierkörper bestimmt, die vor der jeweils nächsten Verabreichung zwischen den Kolben und das Vorschubelement eingebracht wird. Für einen Verwender wird eine einfache, leicht überprüfbare und daher sichere Dosierung ermöglicht.

Bevorzugt ist das Vorschubelement in Vorschubrichtung des Kolbens linear verschiebbar in dem Gehäuse aufgenommen und wird vorzugsweise von einem Knopf bzw. einer Hülse gebildet. Das Dosierelement wird vorzugsweise ebenfalls von einem linear verschiebbaren Körper, beispielsweise einem Knopf bzw. einer Hülse, gebildet. Es ragt vorzugsweise aus dem Gehäuse heraus. Das Vorschubelement und das Dosierelement werden vorzugsweise beide jeweils in einer Ausgangsstellung vorgespannt gehalten. Eine Betätigung des Vorschubelements und des Dosierelements erfolgt entgegen dieser Haltekraft. Ein automatisches Zurückstellen in die Ausgangsstellung wird somit ermöglicht.

Das Dosierelement ist bevorzugt zwischen zwei Endstellungen hin und her bewegbar. Die eine Endstellung ist die Ausgangsstellung. Die andere, vordere Endstellung nimmt das Dosierelement ein, wenn es einen Dosierkörper in die Dosierposition bewegt hat. Die Fixierung des Dosierkörpers in der Dosierposition kann in der vorderen Endstellung einen vorderen Anschlag für das Dosierelement bilden. Es kann jedoch auch ein vorderer Anschlag unmittelbar für das Dosierelement ausgebildet sein. Nach dem Anstoßen an dem vorderen Anschlag, nämlich ein vorderen Anschlag für den Dosierkörper oder einen vorderen Anschlag unmittelbar für das Dosierelement, kann das Dosierelement nur wieder in Richtung auf seine Ausgangsstellung zurückbewegt werden. Eine Dosierung mittels eines hin und her bewegbaren Dosierelements ist besonders sicher.

Es kann eine Vorrichtung vorgesehen sein, die eine Rückstellbewegung des Dosierelements erst dann ermöglicht, wenn ein Dosierkörper vollständig in eine Dosierposition zwischen den Kolben und das Vorschubelement eingebracht worden ist. Dazu kann eine Sicherung verwendet werden, die eine Rückstellbewegung verhindert, bis das Dosierelement eine vordere Endstellung erreicht hat, in der ein Dosierkörper beispielsweise an den vorderen Anschlag, der die Bewegung begrenzt, anstößt. Ein Dosierelement kann auf einer gekrümmten Bahn oder vorzugsweise gerade in die Dosierposition gedrückt oder gezogen werden.

Besonders bevorzugt werden die Dosierkörper quer zur Vorschubrichtung des Kolbens in die Dosierposition zwischen den Kolben und das Vorschubelement bewegt, insbesondere geschoben. Alternativ können die Dosierkörper von einem Vorratsstapel weg in Ausschüttrichtung zwischen den Kolben und das Vorschubelement verlagert werden. Der Vorratsstapel kann insbesondere bei Verlagerung der Dosierkörper in Ausschüttrichtung längs der Längsachse des Reservoirs versetzt angeordnet sein. Vorzugsweise werden die Dosierkörper gegen einen Anschlag in die Dosierposition bewegt. Das Dosierelement drückt bei dem Bewegen eines Dosierkörpers vorzugsweise lose gegen den Dosierkörper, d.h. es besteht zwischen dem Dosierelement und dem jeweiligen Dosierkörper eine Verbindung bevorzugt nur durch das Andrücken gegen den Dosierkörper.

Die Dosierkörper werden vorzugsweise lose in Vorschubrichtung zwischen dem Kolben und dem Vorschubelement übereinandergestapelt. Bevorzugt werden sie in der Dosierposition von dem Gehäuse geführt. Bei der Betätigung der Ausschütteinrichtung werden sie in das Reservoir geschoben und dann vorzugsweise von der inneren Mantelfläche des Reservoirs geführt. Mehrere Dosierkörper werden vorzugsweise in die Dosierposition gebracht, indem sie von der Seite kommend übereinandergeschoben werden. In Verschieberichtung der Dosierkörper können die Dorsierkörper verjüngt sein, um ein Übereinanderschieben der Dosierkörper zu erleichtern.

Als Dosierkörper werden vorzugsweise Plättchen verwendet. Besonders bevorzugt kommen flache Plättchen mit einer geringen Dicke zum Einsatz. Die Summe der Dicken der Dosierkörper, welche zwischen den Kolben und das Vorschubelement eingebracht wird, bestimmt die Weglänge und somit die Dosierung. Die Dicke eines Dosierkörpers ist die geringste einstellbare Weglänge. Die Dosierkörper haben vorzugsweise je die gleiche Dicke. Sie können linsen- oder zylinderförmig sein oder sonstige Formen annehmen. Die Dosierkörper können eine abgeflachte Seite oder zwei abgeflachte Seiten aufweisen. Hierdurch wird eine Geradführung verbessert.

Die Dosierkörper werden für eine Bevorratung vor dem Dosieren bevorzugt in einem Magazin zu einem Dosierkörpervorratsstapel übereinandergestapelt aufgenommen. Das Magazin ist vorzugsweise in das Gehäuse integriert. Bei einer Bewegung des Dosierelements quer zu dem Vorratsstapel verlagert das Dosierelement einen der Dosierkörper von diesem Vorratsstapel weg zwischen den Kolben und das Vorschubelement in die Dosierposition.

Der Vorratsstapel wird vorzugsweise von einer vorgespannten Feder gegen einen Anschlag gedrückt. Eine seitliche Führung des Vorratsstapels wird von dem Magazin übernommen. Der Anschlag und die seitliche Führung wirken vorzugsweise derart zusammen, dass genau ein Dosierkörper für eine Querverschiebung freigegeben ist. Hierfür kann zwischen der seitlichen Führung und dem Anschlag ein Abstand bestehen, oder es ist die seitliche Führung durchbrochen, so dass das Dosierelement in Eingriff mit einem äußersten Dosierkörper des Stapels gebracht werden kann. Nach einer Bewegung dieses Dosierkörpers in die Dosierposition drückt die Feder den Vorratsstapel um eine Position weiter, so dass wieder ein Dosierkörper an dem Anschlag anliegt und für eine Querverlagerung aus dem Vorratsstapel freigegeben ist.

Bevorzugt wird das Magazin seitlich versetzt neben dem Reservoir angeordnet. Besonders bevorzugt liegt es auch axial auf gleicher Höhe mit dem Reservoir.

Die Dosierkörper werden in einer bevorzugten Ausführung quer zu dem Vorratsstapel und quer zur Vorschubrichtung des Kolbens von dem Vorratsstapel weg zwischen den Kolben und das Vorschubelement gerade verschoben. Zum Einbringen zwischen den Kolben und das Vorschubelement werden die Dosierkörper vorteilhafterweise in einer Bahn geführt. Der Anschlag bildet vorzugsweise auch eine Führung für einen Dosierkörper während er von dem Vorratsstapel weg zwischen den Kolben und das Vorschubelement gebracht wird. Der Anschlag führt einen Dosierkörper an seiner Deckfläche.

Während dem Einbringen eines Dosierkörpers zwischen den Kolben und das Vorschubelement werden das Dosierelement und der Anschlag zumindest teilweise übereinander und/oder ineinander geschoben. Das Dosierelement greift, vorzugsweise mit einer Zunge, an einer seitlichen Fläche des Dosierkörpers an, um ihn vom Vorratsstapel wegzubewegen. Besonders bevorzugt ist der Anschlag gabelförmig ausgeführt und hält einen Dosierkörper jeweils nur an seinen seitlichen Randbereichen. Das Dosierelement greift im mittleren Bereich des Dosierkörpers an und wird bei der Verschiebung des Dosierkörpers in den gabelförmigen Anschlag geschoben. Die Zunge des Dosierelements weist vorzugsweise eine größere Dicke auf als ein einzelner Dosierkörper.

Ein oder mehrere Dosierkörper werden in der Dosierposition vorzugsweise vorgespannt gehalten. Die Vorspannkraft ist dabei geringer als die Haftreibkraft des Kolbens im Reservoir. Durch die Vorspannung der Dosierkörper können sich diese nicht in der Dosierposition verkanten. Es kann beispielsweise das Vorschubelement elastisch in Vorschubrichtung gespannt sein und dabei den oder die Dosierkörper, die sich in der Dosierposition befinden, auf den Kolben zu drücken. Besonders bevorzugt wird jedoch ein relativ zu dem Vorschubelement bewegbarer Niederhalter vorgesehen, der von einer elastisch wirkenden Spannkraft in Vorschubrichtung auf den Kolben zu gedrückt und bei einem Einbringen eines Dosierkörpers in die Dosierposition gegen die Spannkraft und entgegen der Vorschubrichtung bewegt wird. Die elastisch wirkende Spannkraft wird vorzugsweise von einer Feder erzeugt, die den Niederhalter in Vorschubrichtung drückt oder zieht. Ein Ende des Niederhalters, das auf einen Dosierkörper drückt, ist bevorzugt derart ausgebildet, dass ein von der Seite her andrückender Dosierkörper den Niederhalter entgegen der auf ihn wirkenden Spannkraft bewegt.

In einer Weiterbildung der Erfindung werden die Dosierkörper, die vor einem Ausschütten in die Dosierposition gebracht werden, in einem Zählwerk gezählt, und es wird ihre Anzahl von einer Anzeige angezeigt. Bevorzugt wird das Zählwerk während einem Ausschüttvorgang wieder auf Null gestellt. Das Zählwerk weist einen oder mehrere Kontakte auf, der oder die von dem Dosierelement und/oder dem Vorschubelement überschoben werden können.

Die Erfindung findet zwar in erster Linie Anwendung bei mobilen Vorrichtungen, wie z.B. Injektionspens, sie kann jedoch auch mit Vorteil bei stationären Vorrichtungen eingesetzt werden.

Nachfolgend wird ein bevorzugtes Ausführungsbeispiel der Erfindung anhand von Zeichnungen erläutert. Dabei offenbarte Merkmale bilden die Erfindung einzeln und auch in den offenbarten Kombinationen weiter. Es zeigen:
- Fig. 1: eine perspektivische, teilgeschnittene Ansicht einer erfindungsgemäßen Vorrichtung,
- Fig. 2: einen Längsschnitt durch die Vorrichtung in einer Ausgangsstellung,
- Fig. 3: einen Längsschnitt der Vorrichtung nach Einstellung einer Dosis und vor einer Ausschüttung,
- Fig. 4: eine Detaildarstellung des Längsschnitts gemäß Fig. 3,
- Fig. 5: einen Längsschnitt der Vorrichtung nach der Ausschüttung der Dosis.

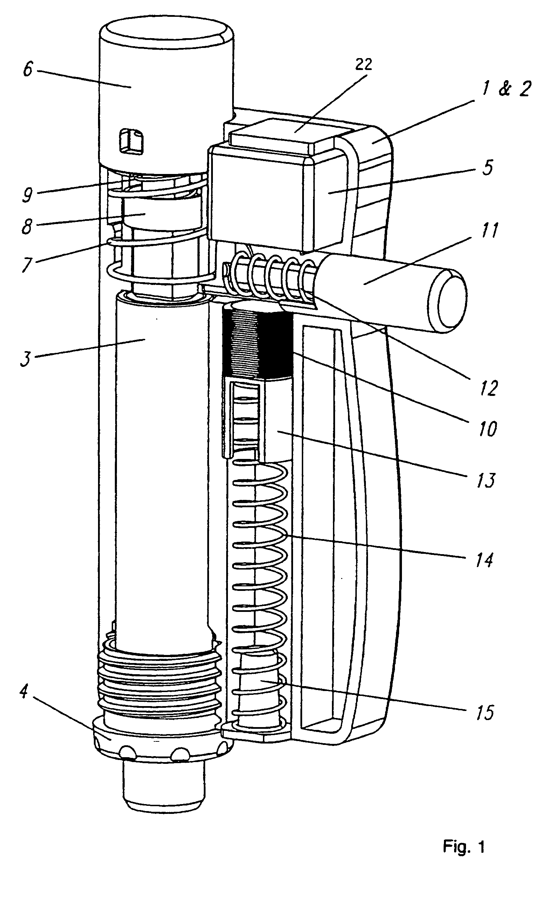

Fig. 1 zeigt eine erfindungsgemäße Vorrichtung in einer dreidimensionalen Ansicht. Ein Gehäuse 1, 2 besteht aus zwei Schalen, von denen in Fig. 1 nur eine dargestellt ist. Die zweite Schale wird symmetrisch zur Schnittachse angeordnet. Im Gehäuse ist eine Ampulle 3 aufgenommen und mittels eines Deckels 4 bzw. Adapters 4 fixiert gehalten. Seitlich parallel versetzt zu der Ampulle 3 sind einzelne Dosierkörper 10 gemeinsam in einem Dosierkörperstapel in einem Magazin 26 im Gehäuse übereinandergestapelt aufgenommen. Die Dosierkörper 10 befinden sich dort in einer Vorratsposition. Oberhalb des Dosierkörperstapels bzw. Vorratsstapels ist eine Dosiereinrichtung mit einem Dosierelement 11 und einem Spannelement 12 angeordnet. Oberhalb der Ampulle 3 ist eine Ausschütteinrichtung mit einem Vorschubelement 6 und einem Spannelement 7 durch das Gehäuse gelagert. Im Gehäuse befinden sich femer ein Zählwerk 5 und eine Anzeige 22, wobei die Anzeige 22 von außen abzulesen ist.

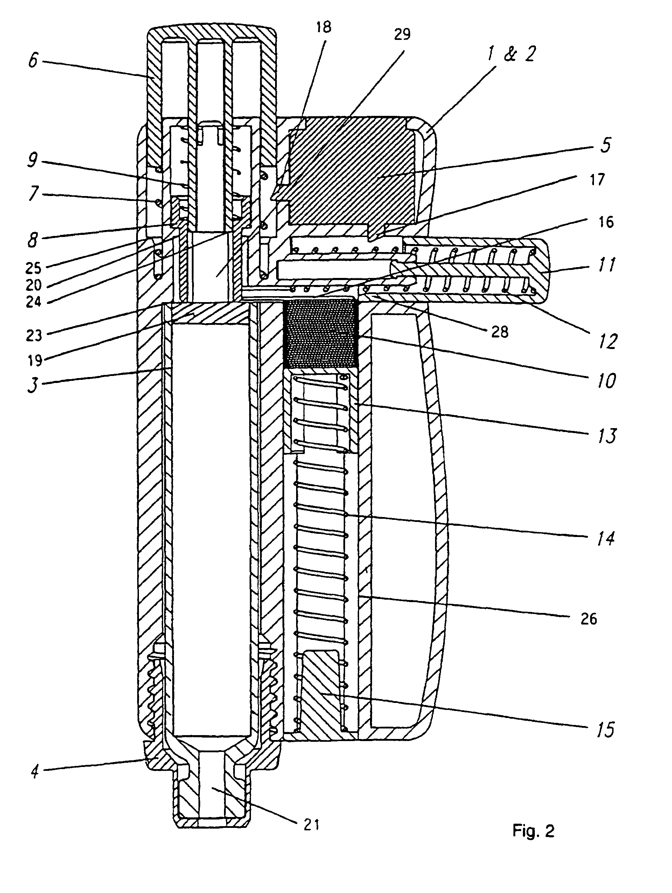

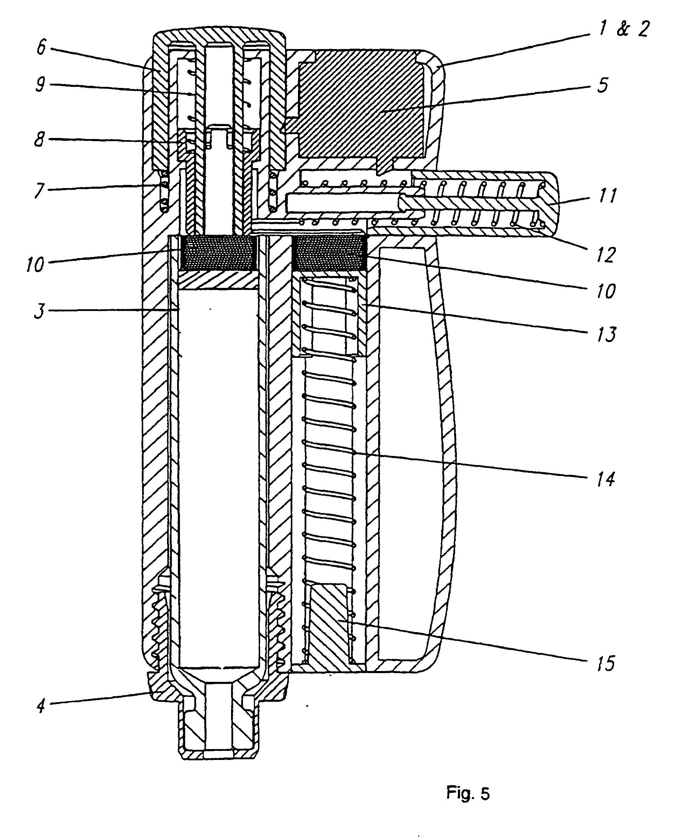

Fig. 2 zeigt die Vorrichtung in einer Ausgangsstellung. Aus der Ampulle 3 wurde noch kein Produktfluid ausgeschüttet. Ein Kolben 19 der bei einer Verschiebung in eine Vorschubrichtung auf einen Auslass 21 der Ampulle 3 zu Produkt aus der Ampulle 3 verdrängt, schließt bündig mit einem oberen Rand 23 der Ampulle 3 ab. Es befindet sich kein Dosierkörper 10 in einer Dosierposition zwischen dem Kolben 19 und dem Vorschubelement 6. Das Vorschubelement 6 wird von dem als Feder ausgebildeten Spannelement 7 in einer Ausgangsstellung gehalten. Von dieser Ausgangsstellung kann das Vorschubelement 6 entgegen der Federkraft linear in Vorschubrichtung auf den Kolben 19 gedrückt werden, bis das Vorschubelement 6 an einem Anschlag 25 des Gehäuses anstößt. In dieser Stellung berührt das Vorschubelement 6 mit einer Druckfläche 24 gerade die Oberseite des Kolbens 19. In dieser Ausschüttstellung des Vorschubelements (vgl. Fig. 5) befindet sich die Druckfläche 24 des Vorschubelements in derselben Ebene wie der Rand 23 der Ampulle 3. Befindet sich ein Dosierkörper 10 zwischen dem Kolben 19 und dem Vorschubelement 6, so wird der Dosierkörper 10 durch das andrückende Vorschubelement 6 in die Ampulle 3 geschoben, wobei der Kolben 19 in Vorschubrichtung gedrückt wird.

Vorzugsweise entspricht die Höhe des Dosierkörpervorratsstapels dem maximalen Vorschubweg des Kolbens 19. In der Zeichnung ist jedoch ein Vorratsstapel geringerer Höhe dargestellt. Ein Führungsstück 13 drückt von einer Unterseite des Vorratsstapels her den Vorratsstapel mit seiner Oberseite auf einen Anschlag 16 zu. Das Führungsstück 13 und die Dosierkörper 10 sind seitlich in dem Magazin 26 geführt. Eine Druckfeder 14, die sich im Magazin 26 zwischen dem Führungsstück 13 und einem Deckel 15 befindet, drückt den Vorratsstapel elastisch nachgiebig den Anschlag 16.

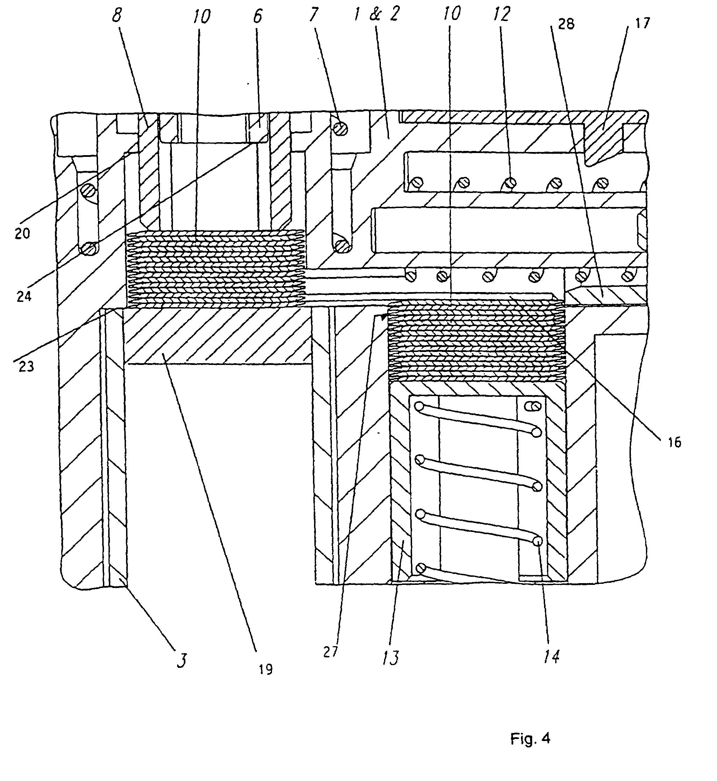

Ein Dosierkörper 10 wird von einem flachen Plättchen gebildet. Das Plättchen weist eine konstante Dicke auf. Die Dosierkörper 10 sind rund und an zwei gegenüberliegenden Seiten abgeflacht. Um ein Übereinanderschieben der Dosierkörper 10 in die Dosierposition zu erleichtern, sind die Dosierkörper 10 an ihren bezüglich ihrer Verschieberichtung vorderen und hinteren Enden angeschrägt. Die Schrägen werden dabei so vorgesehen, dass jeder Dosierkörper 10 leicht unter bereits in der Dosierposition befindliche andere Dosierkörper 10 geschoben werden kann und keine Hemmung auftritt. Die eine Schräge ist von einer oberen Deckfläche der Dosierkörper abfallend ausgebildet, die andere Schräge komplementär dazu von einer unteren Deckfläche aus ansteigend (Fig. 4).

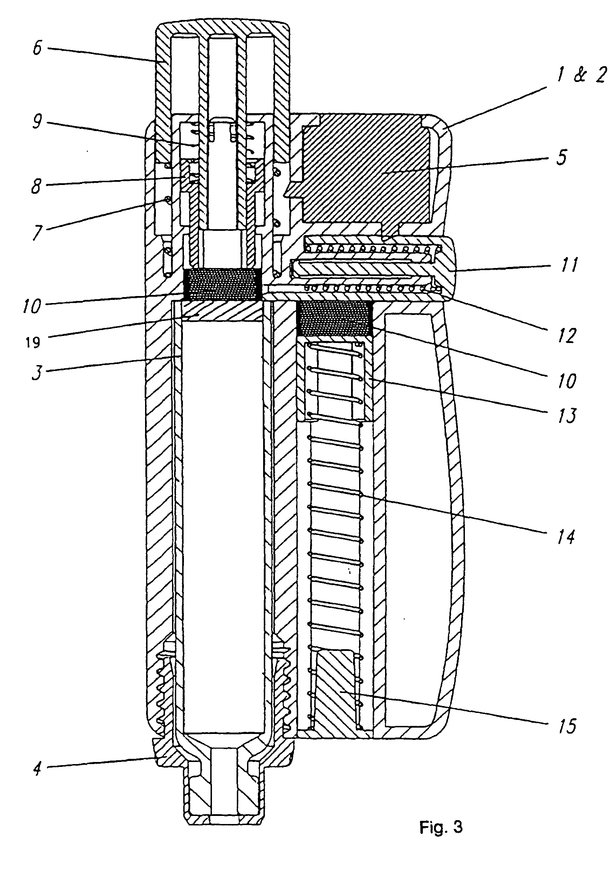

Die Fig. 3 und 4 zeigen die Vorrichtung und ein Detail der Vorrichtung nach der Einstellung der zu verabreichenden Produktdosis und vor deren Ausschüttung. Mehrere der Dosierkörper 10 sind von dem Vorratsstapel in die Dosierposition zwischen dem Kolben 19 und dem Vorschubelement 6 gebracht worden. In den Fig. 3 und 4 ist zu erkennen, dass der Anschlag 16 den Vorratsstapel in einer Position hält, in der nur derjenige Dosierkörper 10 über eine seitliche Führung 27 des Magazins 26 hinaussteht, der an dem Anschlag 16 anliegt. Somit ist nur dieser Dosierkörper 10 für eine Verschiebung entlang des Anschlags 16 freigegeben. Der Anschlag 16 ist gabelförmig ausgeführt. Der Dosierkörper 10 wird an zwei gegenüberliegenden Rändern gegen den Anschlag 16 gedrückt. Bei einem Vorschieben des Dosierelements 11 quer zur Vorschubrichtung des Kolbens 19 greift ein Bereich des Dosierelements 11, der als Schiebezunge 28 ausgebildet ist, an einer Stirnseite des Dosierkörpers 10 an und verschiebt diesen entlang des Anschlags 16. Die Schiebezunge 28 greift dabei in den gabelförmigen Anschlag 16. Das Dosierelement 11 wird in ähnlicher Weise wie das Vorschubelement 6 in einer Ausgangsstellung mittels seines als Druckfeder ausgebildeten Spannelements 12 gehalten.

Ein Dosierkörper 10 wird von dem Dosierelement 11 in einen Dosierraum 29 (Fig. 2) bis auf Anschlag gegen das Gehäuse geschoben. Der Dosierraum 29 ist der Raum zwischen dem Kolben 19 und der Druckfläche 24 des Vorschubelements 6 in seiner Ausgangsstellung. Um in den Dosierraum 29 eingeschobene Dosierkörper 10 gegen die Vorschubrichtung zu fixieren, ist ein Niederhalter 8 elastisch auf den Kolben 19 zu gespannt. Der Niederhalter 8 ist als Hülse ausgebildet, die das Vorschubelement 6 überlappend in und gegen die Vorschubrichtung bewegbar gelagert ist. Die Niederhaltekraft wird von einer Feder 9 erzeugt, wobei die Kraft geringer ist als die Haftkraft des Kolbens 19 in der Ampulle 3. Ein vorderer Bereich des Niederhalters 8, der auf den Kolben 19 drückt, ist an seiner äußeren Mantelfläche angeschrägt ausgeführt. Ein Dosierkörper 10 wird von dem Dosierelement 11 zwischen den Niederhalter 8 und den Kolben 19 geschoben. Der Niederhalter 8 weicht dabei entgegen der Vorschubrichtung des Kolbens 19 und entgegen der Kraft der Feder 9 aus. Der Dosierkörper 10 befindet sich in der Dosierposition zwischen dem Kolben 19 und dem Vorschubelement 6, wobei er von dem Niederhalter 8 vorgespannt gehalten wird.

Im oder neben dem Verschiebeweg des Dosierelements 11 befindet sich ein Schalter bzw. Kontakt 17, der mit einem Zählwerk 5 in Verbindung steht. Wird das Dosierelement 11 an dem Kontakt 17 vorbeigeschoben, schaltet das Zählwerk 5 um eine Zahl weiter. Von einer Anzeige 22 (Fig. 1) wird die Zahl der sich in der Dosierposition befindlichen Dosierkörper 10 angezeigt. Im Verschiebeweg des Vorschubelements 6 befindet sich ein zweiter Schalter bzw. Kontakt 18. Er dient dazu, während einem Ausschüttvorgang das Zählwerk 5 wieder auf Null zu stellen. Bei dem Zählwerk 5 kann es sich um ein elektrisches oder mechanisches Zählwerk handeln.

Im Folgenden wird der Ablauf eines Verabreichungsvorgangs beschrieben. Von der Ausgangsstellung der Vorrichtung gemäß den Fig. 1 und 2 ausgehend werden jeweils einzeln so viele Dosierkörper 10 durch das Dosierelement 11 von dem Vorratsstapel in die Dosierposition zwischen den Kolben 19 und das Vorschubelement 6 gebracht, wie für die gewünschte Dosis benötigt werden. Fig. 3 zeigt die Vorrichtung nach Abschluss des Dosiervorgangs für eine beispielhafte Dosierung. Mehrere Dosierkörper 10 werden in der Dosierposition durch das Gehäuse quer zur Vorschubrichtung in der Flucht zur Ampulle 3 geführt und von dem Niederhalter 8 in bzw. gegen die Vorschubrichtung fixiert. Wird das Vorschubelement 6 in Vorschubrichtung verschoben, drückt es die Dosierkörper 10 aus der Dosierposition in die Ampulle 3 und schiebt dadurch den Kolben 19 in Vorschubrichtung auf den Auslass 21 des Reservoirs 3 zu, so dass die eingestellte Produktdosis aus dem Reservoir 3 verdrängt wird. In Fig. 5 ist die Vorrichtung nach vollendeter Ausschüttung in ihrer Ausschüttstellung vor der selbsttätigen Rückstellung in die Ausgangsstellung dargestellt. Eine dosierte Verabreichung ist beendet und es kann mit einer weiteren Verabreichung begonnen werden, wenn das Vorschubelement 6 durch die Kraft der Feder 7 in seine Ausgangsstellung geschoben wurde.

## Patentansprüche

1. Vorrichtung zur dosierten Verabreichung eines injizierbaren Produkts, umfassend
a) ein Gehäuse (1) mit einem Reservoir (3) für das Produkt,
b) einen Kolben (19), der bei einer Verschiebung in eine Vorschubrichtung auf einen Auslass (21) des Reservoirs (3) zu Produkt aus dem Reservoir (3) verdrängt,
c) eine Ausschütteinrichtung mit einem Vorschubelement (6), das den Kolben (19) um eine eingestellte Weglänge in Vorschubrichtung drückt,
d) und eine Dosiereinrichtung mit einem Dosierelement (11) **dadurch gekennzeichnet, dass** die Dosiereinrichtung mehrere Dosierkörper (10) umfasst, und dass die Weglänge durch wenigstens einen Dosierkörper (10) bestimmt wird, welcher vor dem Betätigen der Ausschütteinrichtung mittels dem Dosierelement (11) zwischen den Kolben (19) und das Vorschubelement (6) eingebracht werden kann.

2. Vorrichtung nach dem vorhergehenden Anspruch, **dadurch gekennzeichnet, dass** die Dosierkörper (10) quer zur Vorschubrichtung des Kolbens (19) zwischen den Kolben (19) und das Vorschubelement (6) geschoben werden.

3. Vorrichtung nach einem der vorhergehenden Ansprüche, **dadurch gekennzeichnet, dass** die Dosierkörper (10) zwischen dem Kolben (19) und dem Vorschubelement (6) lose in Vorschubrichtung des Kolbens (19) übereinander gestapelt werden.

4. Vorrichtung nach einem der vorhergehenden Ansprüche, **dadurch gekennzeichnet, dass** die Dosierkörper (10) Plättchen sind.

5. Vorrichtung nach einem der vorhergehenden Ansprüche, **dadurch gekennzeichnet, dass** die Dosierkörper (10) in Verschieberichtung zum Dosieren vorne und hinten verjüngt sind.

6. Vorrichtung nach einem der vorhergehenden Ansprüche, **dadurch gekennzeichnet, dass** die Dosierkörper (10) in einem Magazin zu einem Vorratsstapel übereinander gestapelt aufgenommen sind
und dass das Dosierelement (11) bei einer Bewegung quer zu dem Vorratsstapel einen Dosierkörper (10) von dem Vorratsstapel weg zwischen den Kolben (19) und das Vorschubelement (6) verlagert.

7. Vorrichtung nach dem vorhergehenden Anspruch, **dadurch gekennzeichnet, dass** der Vorratsstapel von einer vorgespannten Feder (14) gegen einen Anschlag (16) gedrückt wird,
der Vorratsstapel in dem Magazin seitlich geführt ist,
und dass nur ein einziger dem Anschlag (16) nächstgelegener Dosierkörper (10) für eine Querverschiebung freigegeben ist.

8. Vorrichtung nach einem der zwei vorhergehenden Ansprüche, **dadurch gekennzeichnet, dass** das Magazin seitlich versetzt neben dem Reservoir (3) angeordnet ist und die Dosierkörper (10) quer zu dem Vorratsstapel und quer zur Vorschubrichtung des Kolbens (19) von dem Vorratsstapel weg zwischen den Kolben (19) und das Vorschubelement (6) geradverschoben werden.

9. Vorrichtung nach einem der vorhergehenden Ansprüche, **dadurch gekennzeichnet, dass** ein Dosierkörper (10) zum Einbringen zwischen den Kolben (19) und das Vorschubelement (6) entlang eines Anschlags (16) geführt wird.

10. Vorrichtung nach dem vorhergehenden Anspruch, **dadurch gekennzeichnet, dass** das Dosierelement (11) und der Anschlag (16) während dem Einbringen eines Dosierkörpers (10) zwischen den Kolben (19) und das Vorschubelement (6) zumindest teilweise übereinander und/oder ineinander geschoben werden.

11. Vorrichtung nach einem der vorhergehenden Ansprüche, **dadurch gekennzeichnet, dass** ein oder mehrere Dosierkörper (10) in einer Dosierposition zwischen dem Kolben (19) und dem Vorschubelement (6) vorgespannt gehalten werden.

12. Vorrichtung nach einem der vorhergehenden Ansprüche, **dadurch gekennzeichnet, dass** die Ausschütteinrichtung einen Niederhalter (20) umfasst, der von einer elastisch wirkenden Spannkraft in Vorschubrichtung auf den Kolben (19) zu gedrückt wird, und dass der Niederhalter (20) bei einem Einbringen eines Dosierkörpers (10) zwischen den Kolben (19) und das Vorschubelement (6) entgegen der Vorschubrichtung bewegt wird.

13. Vorrichtung nach dem vorhergehenden Anspruch, **dadurch gekennzeichnet, dass** der Niederhalter (20) relativ zu dem Vorschubelement (6) bewegbar angeordnet ist.

14. Vorrichtung nach einem der vorhergehenden Ansprüche, **dadurch gekennzeichnet, dass** die in eine Dosierposition zwischen dem Kolben ( 19) und dem Vorschubelement (6) bewegten Dosierkörper (10) mit einem Zählwerk (5) gezählt werden und ihre Anzahl von einer Anzeige angezeigt wird.

## Claims

1. Device for the dosed administration of an injectable product, comprising
a) a housing (1) with a reservoir (3) for the product,
b) a piston (19) which when pushed in a forward feed direction towards an outlet (21) of the reservoir (3), displaces the product out of the reservoir (3),
c) a dispensing device with a forward feed element (6) which pushes the piston (19) in the forward feed direction by a set path length,
d) and a dosing device with a dosing element (11), **characterised in that** the dosing device comprises several dosing bodies (10), and that the path length is determined by at least one dosing body (10) which, prior to the actuation of the dispensing device can be inserted between the piston (19) and the forward feed element (6), by means of the dosing element (11).

2. Device in accordance with the preceding claim, **characterised in that** the dosing bodies (10) are pushed crosswise to the forward feed direction of the piston (19), between the piston (19) and the forward feed element (6).

3. Device in accordance with one of the preceding claims, **characterised in that** the dosing bodies (10) are stacked loosely above one another in the forward feed direction of the piston (19), between the piston (19) and the forward feed element (6).

4. Device in accordance with of the preceding claims, **characterised in that** the dosing bodies (10) are laminae.

5. Device in accordance with one of the preceding claims, **characterised in that** the dosing bodies (10) are tapered at the front and the back in the pushing direction for dosing.

6. Device in accordance with one of the preceding claims, **characterised in that** the dosing bodies (10) are accommodated in a magazine, stacked on top of one another to form a reserve stack,
and that in the event of a movement cross-wise to the reserve stack, the dosing element (11) displaces a dosing body (10) away from the reserve stack, to between the piston (19) and the forward feed element (6).

7. Device in accordance with the preceding claim, **characterised in that** the reserve stack is pressed against a limit stop (16) by a pre-stressed spring (14),
the reserve stack is held to the side in the magazine,
and that only a single dosing body (10), lying closest to the limit stop (16), is released for being pushed crosswise.

8. Device in accordance with one of the two preceding claims, **characterised in that** the magazine is arranged offset to the side next to the reservoir (3), and the dosing bodies (10) are pushed straight, crosswise to the reserve stack and crosswise to the forward feed direction of the piston (19), away from the reserve stack, to between the piston (19) and the forward feed element (6).

9. Device in accordance with one of the preceding claims, **characterised in that** for insertion between the piston (19) and the forward feed element (6), a dosing body (10) is guided along a limit stop (16).

10. Device in accordance with the preceding claim, **characterised in that** during the insertion of a dosing body (10) between the piston (19) and the forward feed element (6), the dosing element (11) and the limit stop (16) are at least partially pushed over and/or into one another.

11. Device in accordance with one of the preceding claims, **characterised in that** one or more dosing bodies (10) are held in a pre-stressed manner, in a dosing position between the piston (19) and the forward feed element (6).

12. Device in accordance with one of the preceding claims, **characterised in that** the dispensing device comprises a holding-down element (20) which is pressed in a forward feed direction towards the piston (19) by an elastically-acting tension force, and that when a dosing body (10) is inserted between the piston (19) and the forward feed element (6), the holding-down device (20) is moved against the forward feed direction.

13. Device in accordance with the preceding claim, **characterised in that** the holding-down device (20) is arranged so as to be movable relative to the forward feed element (6).

14. Device in accordance with one of the preceding claims, **characterised in that** the dosing bodies (10) which are moved into a dosing position between the piston (19) and the forward feed element (6) are counted with a counter (5) and the number of them is displayed by a display.

## Revendications

1. Dispositif pour l'administration dosée d'un produit injectable, comprenant
a) un boîtier (1) avec un réservoir (3) pour le produit,
b) un piston (19) qui refoule du produit de son réservoir (3) lors d'un déplacement dans un sens d'avancement en direction d'une sortie (21) du réservoir (3),
c) un système de versement avec un élément d'avancement (6), qui appuie le piston (19) d'une longueur de course réglée dans le sens d'avancement,
d) et un système de dosage avec un élément de dosage (11), **caractérisé en ce que** le système de dosage comprend plusieurs corps de dosage (10) et que la longueur de course est déterminée par au moins un corps de dosage (10), qui peut être introduit avant l'actionnement du système de versement au moyen de l'élément de dosage (11) entre le piston (19) et l'élément d'avancement (6).

2. Dispositif selon la revendication précédente, **caractérisé en ce que** les corps de dosage (10) sont poussés transversalement au sens d'avancement du piston (19) entre le piston (19) et l'élément d'avancement (6).

3. Dispositif selon l'une quelconque des revendications précédentes, **caractérisé en ce que** les corps de dosage (10) sont empilés les uns au-dessus des autres entre le piston (19) et l'élément d'avancement (6) librement dans le sens d'avancement du piston (19).

4. Dispositif selon l'une quelconque des revendications précédentes, **caractérisé en ce que** les corps de dosage (6) sont des plaquettes.

5. Dispositif selon l'une quelconque des revendications précédentes, **caractérisé en ce que** les corps de dosage (10) sont rétrécis à l'avant et à l'arrière dans le sens d'avancement pour le dosage.

6. Dispositif selon l'une quelconque des revendications précédentes, **caractérisé en ce que** les corps de dosage (10) sont réceptionnés dans un magasin superposés les uns sur les autres en une pile de réserve
et **en ce que** l'élément de dosage (11) déplace un corps de dosage (10), lors d'un déplacement transversalement à la pile de réserve, en partant de la pile de réserve entre le piston (19) et l'élément d'avancement (6).

7. Dispositif selon la revendication précédente, **caractérisé en ce que** la pile de réserve est poussée par un ressort (14) précontraint contre une butée (16),
la pile de réserve est guidée latéralement dans le magasin,
et **en ce que** seul un unique corps de dosage (10) situé le plus près de la butée (16) est libéré pour un déplacement transversal.

8. Dispositif selon l'une quelconque des revendications précédentes, **caractérisé en ce que** le magasin est décalé latéralement à côté du réservoir (3) et les corps de dosage (10) sont déplacés en ligne droite, transversalement à la pile de réserve et transversalement au sens d'avancement du piston (19), en partant de la pile de réserve entre le piston (19) et l'élément d'avancement (6).

9. Dispositif selon l'une quelconque des revendications précédentes, **caractérisé en ce qu'**un corps de dosage (10) est guidé pour l'introduction entre le piston (19) et l'élément d'avancement (6) le long d'une butée (16).

10. Dispositif selon la revendication précédente, **caractérisé en ce que** l'élément de dosage (11) et la butée (16) sont poussés au moins partiellement les uns au-dessus des autres et/ou les uns dans les autres pendant l'introduction d'un corps de dosage (10) entre le piston (19) et l'élément d'avancement (6).

11. Dispositif selon l'une quelconque des revendications précédentes, **caractérisé en ce qu'**un ou plusieurs corps de dosage (10) sont maintenus précontraints dans une position de dosage entre le piston (19) et l'élément d'avancement (6).

12. Dispositif selon l'une quelconque des revendications précédentes, **caractérisé en ce que** le système de versement comprend un serre-flan (20) qui est appuyé par une force de serrage agissant de façon élastique dans le sens d'avancement en direction du piston (19) et **en ce que** le serre-flan (20) est déplacé dans le sens contraire au sens d'avancement lors d'une introduction d'un corps de dosage (10) entre le piston (19) et l'élément d'avancement (6).

13. Dispositif selon la revendication précédente, **caractérisé en ce que** le serre-flan (20) est disposé de façon mobile par rapport à l'élément d'avancement (6).

14. Dispositif selon l'une quelconque des revendications précédentes, **caractérisé en ce que** les corps de dosage (10) déplacés dans une position de dosage entre le piston (19) et l'élément d'avancement (6) sont comptés avec un compteur (5) et leur nombre est affiché par un dispositif indicateur.
